# EUROPEAN PATENT APPLICATION

(11) **EP 4 239 640 A1**
(43) Date of publication of application: **06.09.2023**
(21) Application number: 22745198.6
(22) Date of filing: 24.01.2022
(51) Int. Cl.: G16C 20/50

(54) **DRUG MOLECULE PROCESSING METHOD AND APPARATUS BASED ON ARTIFICIAL INTELLIGENCE, AND DEVICE, STORAGE MEDIUM AND COMPUTER PROGRAM PRODUCT**

(30) Priority: 28.01.2021 CN 202110119170
(71) Applicant: Tencent Technology (Shenzhen) Company Limited, Shenzhen, Guangdong, 518057 (CN)
(72) Inventor: YU, Yang, Shenzhen, Guangdong 518057 (CN); ZHENG, Liangzhen, Shenzhen, Guangdong 518057 (CN); LIU, Yixuan, Shenzhen, Guangdong 518057 (CN)
(74) Representative: EP&C
(86) International application number: PCT/CN2022/073558
(87) International publication number: WO 2022/161323

(57) **Abstract**

A drug molecule processing method and apparatus based on artificial intelligence, and an electronic device, a computer-readable storage medium and a computer program product, which relate to artificial intelligence technology. The method comprises: determining multiple candidate drug molecules for a target protein; performing activity prediction processing on the basis of the multiple candidate drug molecules and the target protein to obtain activity information of each candidate drug molecule; performing homology modeling processing on the target protein to obtain a reference protein having a homologous structure to the target protein; performing molecular docking processing on the basis of the reference protein and the multiple candidate drug molecules to obtain molecular docking information of each candidate drug molecule; and performing screening processing on the multiple candidate drug molecules on the basis of the activity information of each candidate drug molecule and the molecular docking information of each candidate drug molecule to obtain a target drug molecule for the target protein.

## Description

### RELATED APPLICATION

Embodiments of this application are based upon and claim priority to Chinese Patent Application No. 202110119170.1, filed on January 28, 2021, which is incorporated herein by reference in its entirety.

### FIELD OF THE TECHNOLOGY

This disclosure relates to an intelligent medical technology, and in particular, to an artificial intelligence-based (AI-based) drug molecule processing method and apparatus, an electronic device, a computer-readable storage medium, and a computer program product.

### BACKGROUND OF THE DISCLOSURE

Artificial intelligence (AI) is a comprehensive technology of the computer science, and is to study the design principles and implementation methods of various intelligent machines, so that the machines can perceive, infer, and make decisions. The AI technology is a comprehensive discipline, and relates to wide fields in several major directions such as a natural language processing technology and machine learning (ML)/deep learning (DL). With the development of technologies, the AI technology will be applied to more fields and play an increasingly important role.

Drug screening is a technology for selecting a target drug molecule having reference value for some diseases (for example, polycystic ovarian syndrome) from a large quantity of drug molecules. A large quantity of candidate drug molecules existing in a current compound library may have reference value, but data having reference value is dug out from the large quantity of candidate drug molecules in dependence on manual selection. As a result, neither accuracy nor efficiency is ideal, and costs are quite high.

The related art lacks a solution for efficiently selecting candidate drug molecules based on AI.

### SUMMARY

Embodiments of this disclosure provide an Artificial intelligence (AI)-based drug molecule processing method and apparatus, an electronic device, a computer-readable storage medium, and a computer program product, to select valuable drug molecules efficiently from a large quantity of candidate drug molecules.

The technical solutions of the embodiments of this disclosure are implemented as follows:

An embodiment of this disclosure provides an AI-based drug molecule processing method, including:
determining a plurality of candidate drug molecules for a target protein;
performing activity prediction based on the plurality of candidate drug molecules and the target protein, to obtain activity information of each of the plurality of candidate drug molecules;
performing homology modeling on the target protein, to obtain a reference protein having a structure homologous with that of the target protein;
performing molecular docking based on the reference protein and the plurality of candidate drug molecules, to obtain molecular docking information of each of the plurality of candidate drug molecules; and
selecting a target drug molecule for the target protein from the plurality of candidate drug molecules based on the activity information of each of the plurality of candidate drug molecules and the molecular docking information of each of the plurality of candidate drug molecules

An embodiment of this disclosure provides an AI-based drug molecule processing apparatus, including:
a determining module, configured to determine a plurality of candidate drug molecules for a target protein;
a prediction module, configured to perform activity prediction based on the plurality of candidate drug molecules and the target protein, to obtain activity information of each of the plurality of candidate drug molecules;
a processing module, configured to perform homology modeling on the target protein, to obtain a reference protein having a structure homologous with that of the target protein; and perform molecular docking based on the reference protein and the plurality of candidate drug molecules, to obtain molecular docking information of each of the plurality of candidate drug molecules; and
a selecting module, configured to select a target drug molecule from the plurality of candidate drug molecules based on the activity information of each of the plurality of candidate drug molecules and the molecular docking information of each of the plurality of candidate drug molecules.

An embodiment of this disclosure provides an electronic device for drug molecule processing, including:
a memory, configured to store executable instructions; and
a processor, configured to perform the AI-based drug molecule processing method provided in the embodiments of this disclosure when executing the executable instructions stored in the memory.

An embodiment of this disclosure provides a computer-readable storage medium, storing executable instructions, the executable instructions, when executed by a processor, implementing the AI-based drug molecule processing method provided in the embodiments of this disclosure.

An embodiment of this disclosure provides a computer program product, including a computer program or instruction, the computer program or instruction causing a computer to perform the foregoing AI-based drug molecule processing method.

The embodiments of this disclosure have the following beneficial effects:

A target drug molecule for a target protein is selected from a large quantity of candidate drug molecules based on activity information of the candidate drug molecules and molecular docking information of the candidate drug molecules, thereby ensuring activity of the target drug molecule and a capability of binding the target protein, so as to select a valuable target drug molecule efficiently and automatically.

### BRIEF DESCRIPTION OF THE DRAWINGS

FIG. 1 is a schematic diagram of a G protein-coupled receptor GPR54/Kiss-1 signal pathway provided in the related art.
FIG. 2 is a schematic diagram of structure optimization of a hit compound 1 provided in the related art.
FIG. 3 is a schematic diagram of an application scenario of a medicine system according to an embodiment of this disclosure.
FIG. 4 is a schematic structural diagram of an electronic device for drug molecule processing according to an embodiment of this disclosure.
FIG. 5 to FIG. 7 are schematic flowcharts of artificial intelligence (AI)-based organic matter sample processing methods according to embodiments of this disclosure.
FIG. 8 is a schematic structural diagram of training a prediction model according to an embodiment of this disclosure.
FIG. 9 is a schematic flowchart of deep learning (DL)-based compound activity scoring according to an embodiment of this disclosure.
FIG. 10A and FIG. 10B are schematic structural diagrams of latent active molecules according to an embodiment of this disclosure.

### DESCRIPTION OF EMBODIMENTS

To make the objectives, technical solutions, and advantages of this disclosure clearer, the following describes this disclosure in further detail with reference to the accompanying drawings. The described embodiments are not to be considered as a limitation to this disclosure. All other embodiments obtained by a person of ordinary skill in the art without creative efforts shall fall within the protection scope of this disclosure.

In the following descriptions, the included term "first/second" is merely intended to distinguish similar objects but does not necessarily indicate a specific order of an object. It may be understood that "first/second" is interchangeable in terms of a specific order or sequence if permitted, so that the embodiments of this disclosure described herein can be implemented in a sequence in addition to the sequence shown or described herein.

Unless otherwise defined, meanings of all technical and scientific terms used in this specification are the same as those usually understood by a person skilled in the art to which this disclosure belongs. Terms used in this specification are merely intended to describe objectives of the embodiments of this disclosure, but are not intended to limit this disclosure.

Before the embodiments of this disclosure are further described in detail, terms involved in the embodiments of this disclosure are described. The terms provided in the embodiments of this disclosure are applicable to the following explanations.
1) Deep learning (DL): It is a new research direction in the field of machine learning (ML), and is introduced to ML to cause ML to be closer to the initial goal -artificial intelligence (AI). DL is learning the inherent law and the hierarchical representation of sample data, and the information obtained in the learning process is of great help to explanation of data such as texts, images and sounds. The ultimate goal of DL is enabling a machine to have an analytic learning capability and recognize data such as texts, images and sounds like a human being.
2) Convolutional neural network (CNN): as a feedforward neural network (FNN) that contains convolution calculation and has a deep structure, CNN is one of representative algorithms of deep learning. The convolutional neural network has a representation learning capability, and can perform shift-invariant classification on inputted images according to its hierarchical structure.
3) Simplified Molecular Input Line Entry Specification (SMILES): It is a specification in which a molecular structure is explicitly described using an American Standard Code for Information Interchange (ASCII) character string. A SMILES expression may describe a three-dimensional chemical structure using a string of characters, for example, a SMILES expression of cyclohexane (C6H12) is C1CCCCC1, that is, C1CCCCC1 represents cyclohexane.
4) Drug molecule: It is a chemical structure of a drug, for example, a benzene ring is a drug molecule.
5) Drug property: It represents a property of a molecular structure of a drug, for example, solubility, blood-brain barrier permeability and toxicity (if a type of drug molecules can cause direct or indirect damage after contacting a life organism or entering a biological living body, the type of drug molecules has biological toxicity).
6) Molecular docking: It is one of important methods for molecular simulation, is a method for performing drug design through a feature of a receptor and interaction between the receptor and a drug molecule, and is essentially a recognition process between a plurality of molecules, where the process involves space matching and energy matching between molecules. In the field of drug research and development, it is mainly used for calculating and viewing a binding manner of a small molecule and a target spot protein and a key binding effect. The molecular docking method is simplified to some extent, and may be divided into rigid docking, semi-flexible docking and flexible docking according to extents and manners of simplification.
7) Homology modeling: A three-dimensional structure of a protein is important information for understanding its biological and physiological functions and performing drug design based on a target spot structure. The homology modeling is a method starting from an amino acid sequence of a protein, and constructing a three-dimensional structure of a target protein using a three-dimensional structure of an experimentally analyzed homologous protein as a template.
8) Binding pocket: In drug design, a cavity where a small molecule and a protein are bound to play a role in regulating a function of the protein is referred to as a binding pocket.
9) G protein-coupled receptor (GPCR): It is a collective name of a major type of membrane protein receptors. A common point of this type of receptors is that a three-dimensional structure of each receptor has seven transmembrane α helices, and each of a C-terminal of its peptide chain and a cytoplasmic loop connecting the fifth and sixth transmembrane helices has a G protein binding site.
10) Lead compound: It is briefly referred to as a leader, is a compound having a biological activity and a chemical structure and obtained through various approaches and means, and is used for further compound structure reconstruction.

The polycystic ovarian syndrome (PCOS) is a disease caused hormone disharmony, and the specific reason which causes this disharmony has not been known yet. An ovary generates estrogens and progesterones, which are female hormones. The ovary also generates androgens, which are male hormones. In the polycystic ovarian syndrome, the ovary generates excess androgens, which causes a hormone disharmony. The hormone disharmony may have wide effects on the human body, and these effects may be slight or severe, and affect about one tenth of women. Like any chronic disease, the polycystic ovarian syndrome affects life to an extent, but if not treated, the polycystic ovarian syndrome has a risk of causing other health problems, including: heart disease, diabetes, obesity, endometrial cancer, infertility and the like.

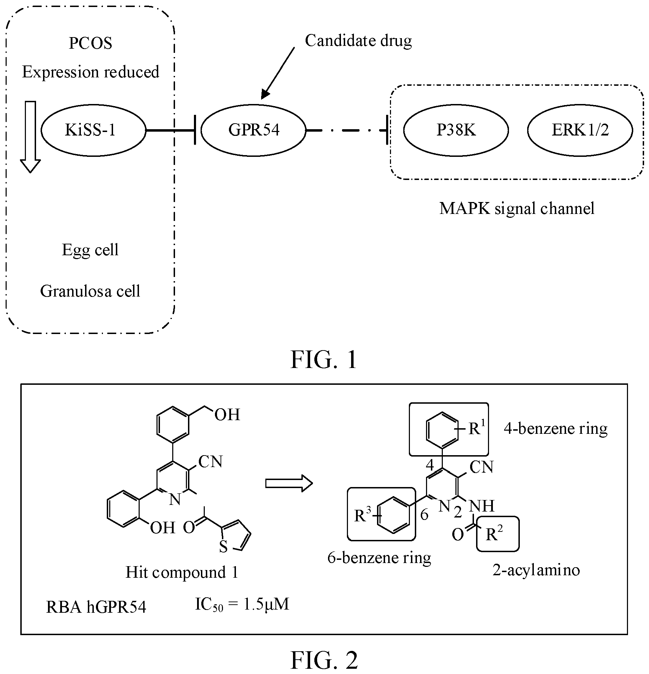

In the related art, the PCOS is treated mainly by increasing the level of female hormones or reducing the effect of increased male hormones through hormone treatment. There has not been a drug mainly targeted at such a disease as polycystic ovarian syndrome on the market by now. As shown in FIG. 1, an abnormal MAPK or ERK signal channel causes a defect of a metabolism signal of a PCOS patient and excessive secretion of androgens of an ovary, a G protein-coupled receptor GPR54/Kiss-1 has a specific effect on a reproductive system syndrome, and the expression level of several key proteins in the ovary can be significantly affected by binding of a polypeptide molecule Kisspeptin (a type of hormones generated by a Kp neuron, where a reproductive activity is regulated by adjusting the content of estrogens in a living body) and a GPR54 protein, thereby alleviating the polycystic ovarian syndrome.

Although Kisspeptin can be bound to GPR54 to alleviate PCOS, because Kisspeptin is highly expressed in a central nervous system, PCOS cannot be treated by directly injecting Kisspeptin. Therefore, search for a small molecule that can be selectively bound to the GPR54 protein and meanwhile does not generate a toxic side-effect on the central nervous system through the blood-brain barrier is quite promising in research and clinically valuable.

However, very few drug molecules are developed for the GPR54 target spot due to main reasons including: 1) the crystal structure of the target spot GPR54 is unpredictable information, which is very difficult to implement for structure-based drug molecule design; and 2) data of related active molecules is relatively little. As shown in FIG. 2, molecules are optimized according to a structure activity relationship through a design-synthesis-test-redesign cyclic process to intend to find a lead compound (hit compound 1) with ideal activity, where the activity is 1.2 µM.

However, the foregoing drug design policy needs to consume a large quantity of manpower and material resources to continuously make trial-and-error attempts. Therefore, not only the efficiency is low and the period is long, but also the accuracy is relatively low. Moreover, in this process, the blood-brain barrier permeability and other absorption, distribution, metabolism, excretion and toxicity (ADMET properties) of drugs need to be continuously tested, so as to ensure safety and physicochemical properties of drug molecules and in-vivo metabolism stability.

To resolve the foregoing problems, the embodiments of this disclosure provide an AI-based drug molecule processing method and apparatus, an electronic device, a computer-readable storage medium, and a computer program product, to select a valuable target drug molecule efficiently from a large quantity of candidate drug molecules.

The AI-based drug molecule processing method provided in the embodiments of this disclosure may be implemented by a terminal or a server alone; or may be implemented by a terminal and a server collaboratively. For example, the terminal alone undertakes the following AI-based drug molecule processing method, or the terminal sends a selection request for drug molecules (including information about a target protein) to the server, the server performs the AI-based drug molecule processing method according to the received selection request for drug molecules, to obtain a target drug molecule for the target protein, and a medicine research and development person may quickly perform subsequent drug molecule research, analysis and the like according to the selected target drug molecule.

The electronic device for drug molecule processing provided in the embodiments of this disclosure may be various types of terminal devices or servers. The server may be an independent physical server, or may be a server cluster composed of a plurality of physical servers or a distributed system, or may be a cloud server that provides cloud computing services. The terminal may be a smartphone, a tablet computer, a notebook computer, a desktop computer, a smart speaker, a smartwatch, or the like, but is not limited thereto. The terminal and the server may be directly or indirectly connected in a wired or wireless communication manner. This is not limited in this disclosure.

The servers are used as an example, and may be, for example, a server cluster deployed in the cloud, to open an AI cloud service (AIaaS, AI as a Service) to users, an AIaaS platform splits several types of common AI services, and provides an independent or packaging service in the cloud, this service mode is similar to an AI theme shopping mall, and all users may access and use, through an application programming interface, one or more AI services provided by the AIaaS platform.

For example, one of the AI cloud services may be a drug molecule processing service, that is, a drug molecule processing program provided in the embodiments of this disclosure is encapsulated in the server in the cloud. A user invokes a drug molecule processing service in the cloud service through a terminal (on which a client is run, for example, a drug selection client), so that the server deployed in the cloud invokes the encapsulated drug molecule processing program, selects a target drug molecule from a plurality of candidate drug molecules based on activity information of the candidate drug molecules and molecular docking information of the candidate drug molecules, subsequently obtains a compound library for GPCR based on the target drug molecule in response to a selection request for drug molecules, for example, for a drug selection application, and selects a target drug molecule for GPCR from a plurality of candidate drug molecules in the compound library based on activity information of the candidate drug molecules and molecular docking information of the candidate drug molecules. Because a G protein-coupled receptor has a specific effect on a reproductive system syndrome, a drug research and development person may quickly obtain drug molecules having a positive effect on the reproductive system syndrome from the target drug molecule subsequently.

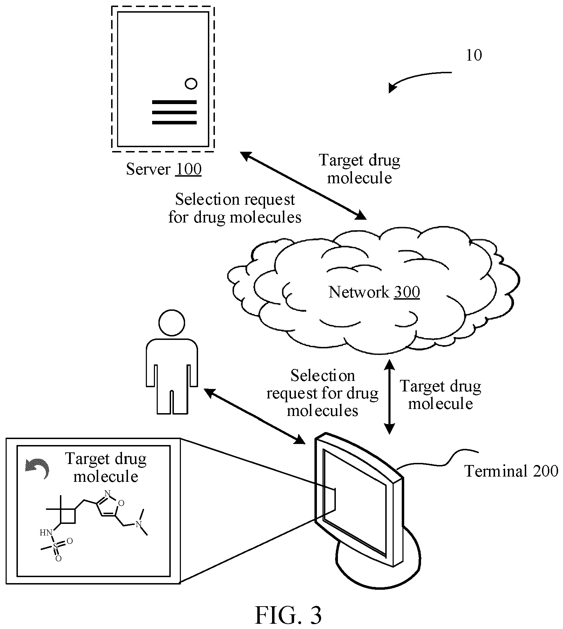

FIG. 3 is a schematic diagram of an application scenario of a medicine system 10 according to an embodiment of this disclosure. A terminal 200 is connected to a server 100 by using a network 300. The network 300 may be a wide area network or a local area network, or a combination thereof.

The terminal 200 (on which a client is run, for example, a drug selection client) may be used for obtaining a target protein and a compound library (including a plurality of candidate drug molecules). For example, a drug research and development person inputs, through an input interface of the terminal 200, the target protein (for example, GPCR) and the compound library (for example, ZINC library) used for performing drug selection, so as to obtain the target protein and the compound library, and automatically generate a selection request for drug molecules.

In some embodiments, a drug molecule processing plug-in may be implanted in the client run in the terminal, and is used for implementing an AI-based drug molecule processing method in the client locally. For example, after obtaining the selection request for drug molecules (including the compound library for the target protein), the terminal 200 invokes the drug molecule processing plug-in, so as to implement the AI-based drug molecule processing method, to select a target drug molecule from a plurality of candidate drug molecules based on activity information of the candidate drug molecules and molecular docking information of the candidate drug molecules, and subsequently respond to the selection request for drug molecules based on the target drug molecule.

In some embodiments, after obtaining the selection request for drug molecules, the terminal 200 invokes a drug molecule processing interface (which may be provided in the form of a cloud service, that is, drug molecule processing service) of the server 100, and the server 100 selects a target drug molecule from a plurality of candidate drug molecules based on activity information of the candidate drug molecules and molecular docking information of the candidate drug molecules, and subsequently respond to the selection request for drug molecules based on the target drug molecule. For example, for drug selection application, the drug research and development person inputs, through the input interface of the drug selection client, the target protein (for example, GPCR) and the compound library used for performing drug selection (for example, ZINC library), so as to obtain the target protein and the compound library, and automatically generate the selection request for drug molecules; and invoke the drug molecule processing interface of the server 100, and select a target drug molecule for GPCR from a plurality of candidate drug molecules based on activity information of the candidate drug molecules and molecular docking information of the candidate drug molecules in the compound library. Because a G protein-coupled receptor has a specific effect on a reproductive system syndrome, a drug research and development person may quickly obtain drug molecules having a positive effect on the reproductive system syndrome from the target drug molecule subsequently.

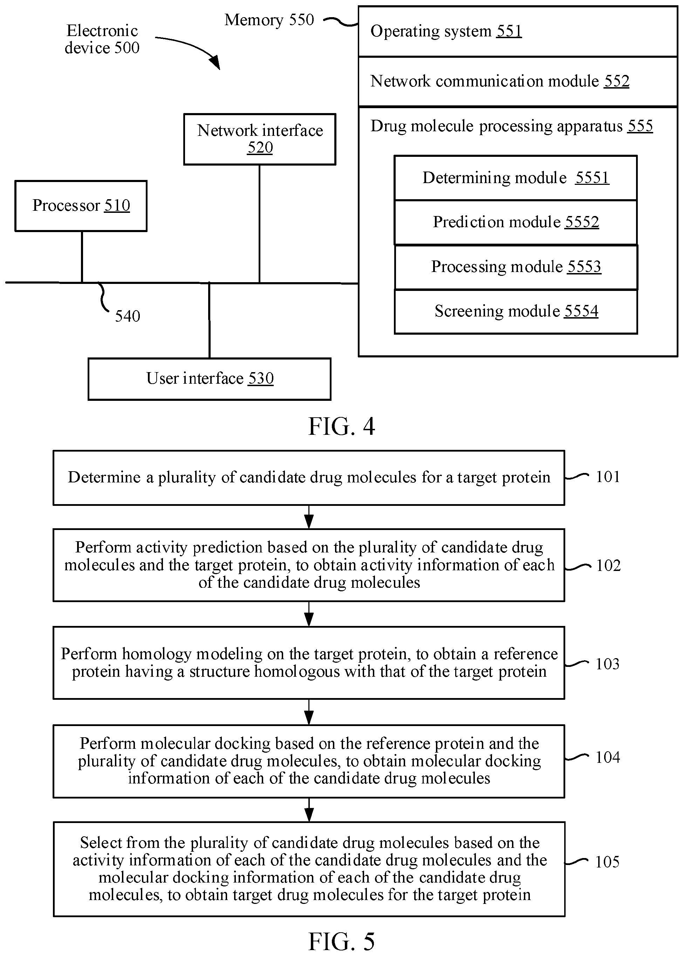

A structure of an electronic device for drug molecule processing provided in an embodiment of this disclosure is described below. FIG. 4 is a schematic structural diagram of an electronic device 500 for drug molecule processing according to an embodiment of this disclosure. A description is made using an example in which the electronic device 500 is a server, and the electronic device 500 for organic matter sample processing shown in FIG. 4 includes: at least one processor 510, a memory 550, at least one network interface 520, and a user interface 530. All the components in the electronic device 500 are coupled together by using a bus system 540. It may be understood that, the bus system 540 is configured to implement connection and communication between the components. In addition to a data bus, the bus system 540 further includes a power bus, a control bus, and a state signal bus. However, for ease of clear description, all types of buses in FIG. 4 are marked as the bus system 540.

The processor 510 may be an integrated circuit chip having a signal processing capability, for example, a general purpose processor, a digital signal processor (DSP), or another programmable logic device (PLD), discrete gate, transistor logical device, or discrete hardware component. The general purpose processor may be a microprocessor, any conventional processor, or the like.

The memory 550 includes a volatile memory or a non-volatile memory, or may include both a volatile memory and a non-volatile memory. The non-volatile memory may be a read-only memory (ROM). The volatile memory may be a random access memory (RAM). The memory 550 described in this embodiment of this disclosure is to include any other suitable type of memories. The memory 550 includes one or more storage devices away from the processor 510 in physical position.

In some embodiments, the memory 550 may store data to support various operations. Examples of the data include a program, a module, and a data structure, or a subset or a superset thereof, which are described below by using examples.

An operating system 551 includes a system program configured to process various basic system services and perform a hardware-related task, such as a framework layer, a core library layer, or a driver layer, and is configured to implement various basic services and process a hardware-based task.

A network communication module 552 is configured to reach another computing device through one or more (wired or wireless) network interfaces 520. Exemplary network interfaces 520 include: Bluetooth, wireless compatible authentication (Wi-Fi), a universal serial bus (USB), and the like.

In some embodiments, a drug molecule processing apparatus provided in the embodiments of this disclosure may be implemented using software, and may be, for example, the drug molecule processing plug-in in the terminal described above, or the drug molecule processing service in the server described above. Certainly, this disclosure is not limited thereto, and the drug molecule processing apparatus provided in the embodiments of this disclosure may be provided as various software embodiments, including various forms of application, software, software module, script or code.

FIG. 4 shows a drug molecule processing apparatus 555 that is stored in the memory 550 and that may be software in the form of a program or a plug-in, for example, a drug molecule processing plug-in and includes a plurality of of modules, including a determining module 5551, a prediction module 5552, a processing module 5553 and a selecting module 5554. The determining module 5551, the prediction module 5552, the processing module 5553 and the selecting module 5554 are configured to implement a drug molecule processing function provided in the embodiments of this disclosure.

As described above, the AI-based drug molecule processing method provided in the embodiments of this disclosure may be implemented by various types of electronic devices. FIG. 5 is a schematic flowchart of an AI-based drug molecule processing method according to an embodiment of this disclosure. A description is made with reference to steps shown in FIG. 5.

In the following steps, a target protein represents a protein having an effect or impact on a disease, for example, a G protein-coupled receptor GPR54/Kiss-1 having a specific effect on a reproductive system syndrome.

Step 101. Determine a plurality of candidate drug molecules for a target protein.

As an example of obtaining the candidate drug molecules, a user may obtain, through an input interface of a terminal, a target protein (for example, GPCR) and a compound library (for example, ZINC library) used for performing drug selection, so as to obtain the target protein and the compound library, and automatically generate a selection request for drug molecules; and sends the selection request to a server. The server parses the selection request for drug molecules, to obtain the target protein and the compound library, reads a plurality of candidate drug molecules for the target protein from the compound library, so as to subsequently perform selection based on the plurality of candidate drug molecules to obtain a target drug molecule having an effect on the target protein.

In some embodiments, the determining a plurality of candidate drug molecules for a target protein includes: selecting a plurality of compounds from compounds in a compound library based on the target protein; and pre-processing the plurality of selected compounds, to obtain the candidate drug molecules for the target protein.

For example, because mass compounds exist in the compound library, a large quantity of calculations need to be consumed, coarse selection may be performed on the compounds in the compound library based on the target protein, so as to obtain candidate drug molecules that may have an effect on the target protein, and the number of candidate drug molecules is reduced, so as to improve efficiency of subsequent drug selection.

In some embodiments, the selecting a plurality of compounds from compounds in a compound library based on the target protein includes: performing Lipinski's Rule of Five-based selection on the compounds in the compound library based on the target protein, to obtain a plurality of compounds obeying the Lipinski's Rule of Five; and deduplicating the plurality of compounds obeying the Lipinski's Rule of Five, to obtain the plurality of selected compounds.

For example, Lipinski's Rule of Five-based selection is first performed on the compounds in the compound library based on the target protein, to obtain compounds obeying the Lipinski's Rule of Five, that is, compounds having pharmacological activity or biological activity, and then the compounds obeying the Lipinski's Rule of Five are deduplicated, to reduce the number of compounds again. The Lipinski's Rule of Five is used for evaluating whether a compound can be used as a drug molecule (not all compounds can be used as drug molecules). For example, the Lipinski's Rule of Five is: 250 ≤ molecular weight ≤ 750; -2 ≤ logarithmic value of an oil-water partition coefficient of a compound ≤ 7; hydrogen bond acceptor +hydrogen bond donor <10; the number of rotatable bonds <10; and topological polar surface area <150.

In some embodiments, the pre-processing the plurality of selected compounds, to obtain the candidate drug molecules for the target protein includes: chemically filtering the plurality of selected compounds based on a target group, to obtain a plurality of filtered compounds; and removing an enantiomer of a chiral compound from the plurality of filtered compounds, to obtain the candidate drug molecules for the target protein.

The target group represents a group undesired in medicinal chemistry. For example, a molecule containing toxicophore groups and reactive groups, that is, a compound containing the target group of the selected compounds is removed, to perform medicinal chemistry filtering. Because the compound library contains enantiomers of many chiral compounds, these chiral compounds are the same in molecular weight and molecular structure, but opposite in left and right arrangement, that is, similar in effect, the enantiomers of the chiral compounds may be removed, so as to reduce the number of candidate drug molecules, and reduce the amount of subsequently calculating the candidate drug molecules.

Step 102. Perform activity prediction based on the plurality of candidate drug molecules and the target protein, to obtain activity information of each of the candidate drug molecules.

For example, after the plurality of candidate drug molecules are obtained, activity prediction is performed on each candidate drug molecule and the target protein, to obtain an activity score (activity information) of each candidate drug molecule, and activity of the candidate drug molecule is evaluated through the activity score, so as to subsequently perform drug selection based on the activity information.

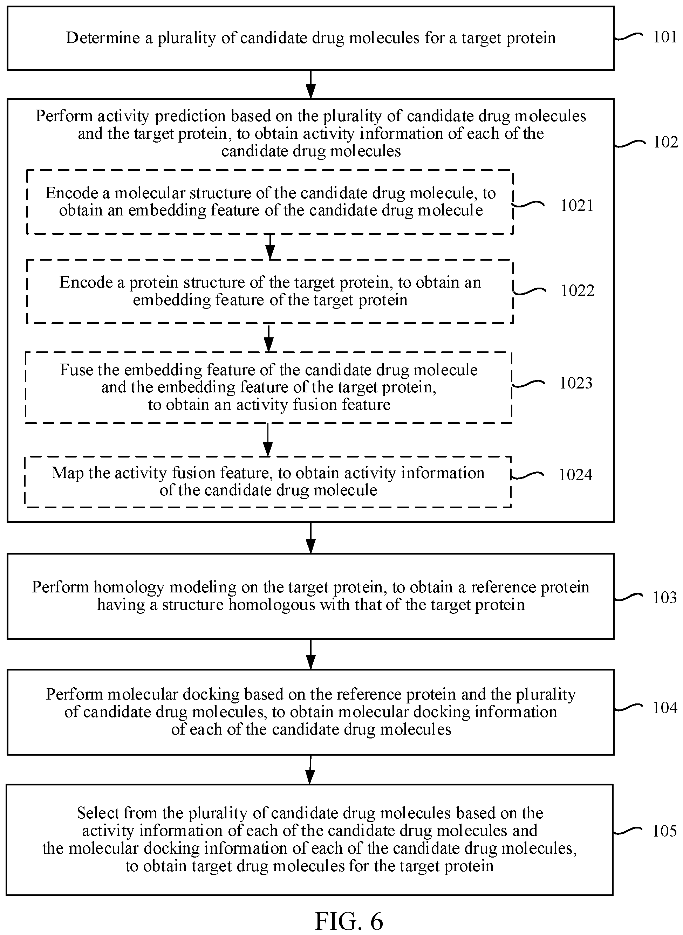

FIG. 6 is a schematic flowchart of an AI-based drug molecule processing method according to an embodiment of this disclosure. As shown in FIG. 6, step 102 in FIG. 5 may be implemented by step 1021 to step 1024: Step 1021. Perform the following processing for any one of the plurality of candidate drug molecules: encoding a molecular structure of the candidate drug molecule, to obtain an embedding feature of the candidate drug molecule. Step 1022. Encode a protein structure of the target protein, to obtain an embedding feature of the target protein. Step 1023. Fuse the embedding feature of the candidate drug molecule and the embedding feature of the target protein, to obtain an activity fusion feature. Step 1024. Map the activity fusion feature, to obtain activity information of the candidate drug molecule.

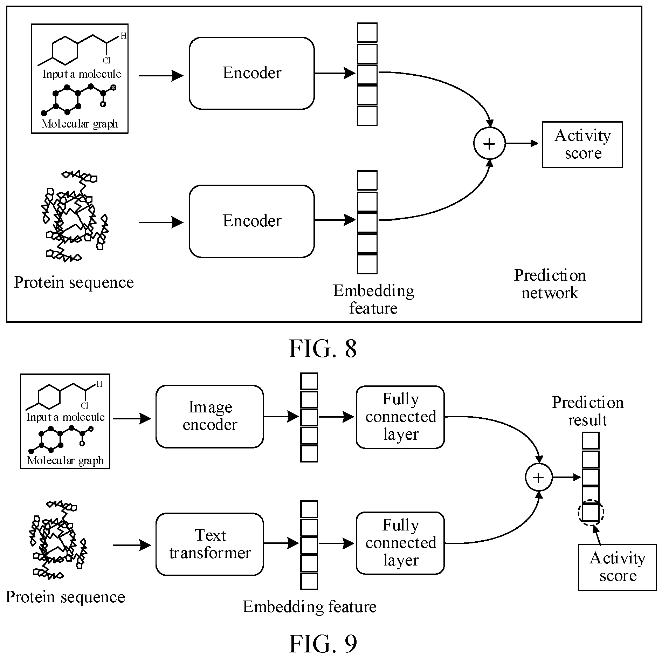

For example, as shown in FIG. 8, through an encoder of a prediction network, a molecular structure (molecular graph) of a candidate drug molecule is encoded to obtain an embedding feature of the candidate drug molecule, and a protein structure (protein sequence) of a target protein is encoded to obtain an embedding feature of the target protein; and then the embedding feature of the candidate drug molecule and the embedding feature of the target protein are fused, and activity fusion feature obtained by the fusion is mapped, to obtain an activity score of the candidate drug molecule, thereby learning activity of the candidate drug molecule through an AI technology, so as to subsequently perform selection based on the accurate activity of the candidate drug molecule.

Continuing from the foregoing example, a molecular graph of the candidate drug molecule is constructed based on the molecular structure of the candidate drug molecule; and image encoding is performed on the molecular graph of the candidate drug molecule through an image encoder (for example, DMPNN), to obtain the embedding feature of the candidate drug molecule, thereby obtaining the accurate embedding feature of the candidate drug molecule through the image encoding.

Continuing from the foregoing example, a protein sequence of the target protein is determined based on the protein structure of the target protein; and the protein sequence of the target protein is encoded in a text transformation form through a text transformer (for example, Doc-to-Vector), to obtain the embedding feature of the target protein, thereby obtaining the accurate embedding feature of the target protein through text encoding.

Continuing from the foregoing example, the fusing the embedding feature of the candidate drug molecule and the embedding feature of the target protein, to obtain an activity fusion feature includes the following 3 manners:
Manner 1. Sum the embedding feature of the candidate drug molecule and the embedding feature of the target protein, and use a summing result as the activity fusion feature.
Manner 2. Concatenate the embedding feature of the candidate drug molecule and the embedding feature of the target protein, and use a concatenating result as the activity fusion feature.
Manner 3. Map the embedding feature of the candidate drug molecule and the embedding feature of the target protein, to obtain an intermediate feature vector including the candidate drug molecule and the target protein; and perform affine transformation on the intermediate feature vector, to obtain the activity fusion feature.

The fusion manners of the manner 1 and the manner 2 are relatively simple, and may save the fusion calculation amount, and the fusion manner of the manner 3 is relatively precise, and may perform accurate fusion, so as to obtain the accurate activity fusion feature, and subsequently perform accurate activity prediction.

Continuing from the foregoing example, the mapping the activity fusion feature, to obtain activity information of the candidate drug molecule includes: mapping the activity fusion feature to a latent vector space, to obtain a latent vector of the activity fusion feature; and performing nonlinear mapping on the latent vector of the activity fusion feature, and using an obtained activity mark of the candidate drug molecule as the activity information of the candidate drug molecule.

For example, the activity fusion feature is mapped to the latent vector space through a fully connected layer in the prediction network, to obtain a latent vector of the activity fusion feature; and then nonlinear mapping is performed on the latent vector of the activity fusion feature through an activation layer in the prediction network, and an obtained activity mark (activity score) of the candidate drug molecule is used as the activity information of the candidate drug molecule.

Step 103. Perform homology modeling on the target protein, to obtain a reference protein having a structure homologous with that of the target protein.

Because information about the crystal structure of the target protein (for example, GPR54 protein) in the embodiments of this disclosure is unpredictable information, and cannot be molecularly docked based on the target protein, it is necessary to use a homology modeling method to establish sequence information of a reference protein having a structure homologous with that of the target protein, that is, protein having a structure homologous with that of the target protein.

In some embodiments, the performing homology modeling on the target protein, to obtain a reference protein having a structure homologous with that of the target protein includes: performing the following processing for any candidate protein in a protein library: performing similarity processing on a sequence of the candidate protein and a sequence of the target protein, to obtain a similarity between the candidate protein and the target protein; and performing structure optimization based on a three-dimensional structure of the candidate protein in a case that the similarity is greater than a similarity threshold, to obtain the reference protein having the structure homologous with that of the target protein.

For example, when sequences of two proteins are similar, structures of the two proteins are similar, that is, two protein sequences in a homology relationship (homology structure) have similar structures. First, any protein (a protein with a known structure) in a protein library (including a plurality of candidate proteins) is used as a candidate protein (template protein), and a sequence of the candidate protein and a sequence of the target protein are compared, to obtain a similarity between the candidate protein and the target protein. When the similarity is greater than a similarity threshold, structure optimization is performed based on a three-dimensional structure of the candidate protein, that is, the candidate protein is used as a prototype, a main chain structure model of the target protein is constructed, a vacant region is formed during the comparison between the candidate protein and the target protein, loop modeling is used for obtaining the complete main chain structure model, and a side chain of the model is constructed and optimized, so as to optimize the entire structure model, thereby obtaining a sequence of a reference protein having a structure homologous with that of the target protein.

Step 104. Perform molecular docking based on the reference protein and the plurality of candidate drug molecules, to obtain molecular docking information of each of the candidate drug molecules.

After obtaining the structure of the reference protein, molecular docking is performed based on the reference protein and each candidate drug molecule, to obtain a molecular docking score of each candidate drug molecule and the reference protein; and the molecular docking score of the candidate drug molecule and the reference protein is used as molecular docking information of the candidate drug molecule, and a binding inhibitory effect of the candidate drug molecule is evaluated based on the molecular docking information, so as to subsequently perform accurate drug selection based on the molecular docking information.

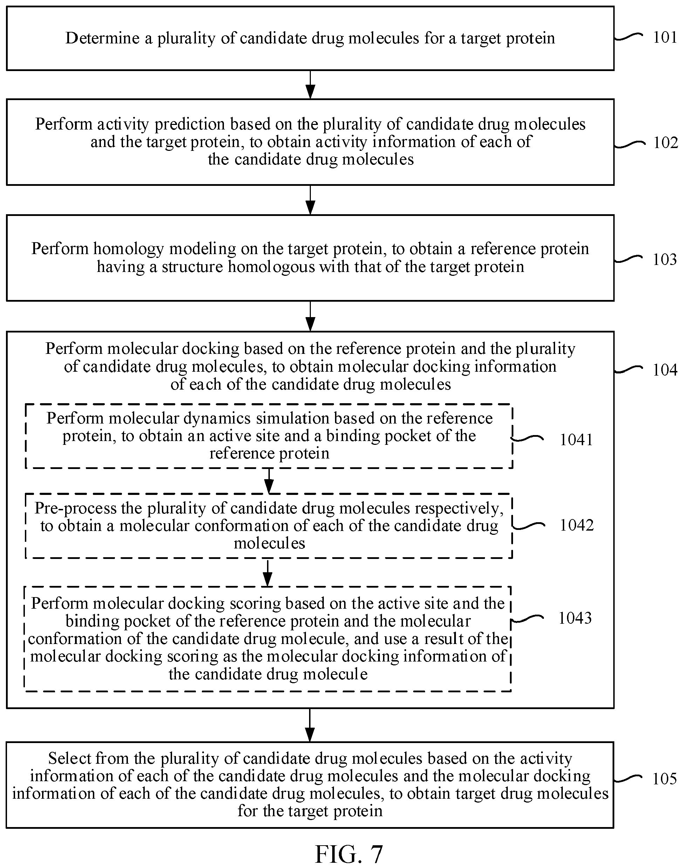

FIG. 7 is a schematic flowchart of an AI-based drug molecule processing method according to an embodiment of this disclosure. As shown in FIG. 7, step 104 in FIG. 5 may be implemented by step 1041 to step 1043: Step 1041. Perform molecular dynamics simulation based on the reference protein, to obtain an active site and a binding pocket of the reference protein. Step 1042. Pre-process the plurality of candidate drug molecules respectively, to obtain a molecular conformation of each of the candidate drug molecules. Step 1043. Perform the following processing for the molecular conformation of each of the candidate drug molecules: performing molecular docking scoring based on the active site and the binding pocket of the reference protein and the molecular conformation of the candidate drug molecule, and using a result of the molecular docking scoring as the molecular docking information of the candidate drug molecule.

For example, a ligand-free binding reference protein structure is simulated through molecular dynamics, a reference protein is inserted into a double-layered membrane structure (POPE), a simulation space is filled with water molecules, salt ions (NaCl) with the concentration of 0.15 M are added, and simulation is performed for more than 100 ns at the temperature of 300 K in a constant pressure system. Reference proteins on a simulation trajectory in last 80 ns in the foregoing simulation system are clustered, and the last clustering-center structure is taken as a molecular docking receptor structure (active site). By using the FTMap method (that is, molecular docking method), a series of chemical groups or molecules (for example, benzene, isobutanol, uric acid and ethanol) are docked to a pocket region, and may be enriched to a region with a plurality of chemical molecules, that is, formed into a pocket region (binding pocket) for molecular docking in a next step.

Continuing from the foregoing example, the pre-processing the plurality of candidate drug molecules respectively includes: performing format transformation on the plurality of candidate drug molecules respectively, to obtain a transformation format of each of the candidate drug molecules; constructing a three-dimensional conformation of each of the candidate drug molecules based on the transformation format of each of the candidate drug molecules; determining a hydrogen atom addible position of each of the candidate drug molecules based on the three-dimensional conformation of each of the candidate drug molecules; and adding a hydrogen atom to the hydrogen atom addible position, to obtain the molecular conformation of the candidate drug molecule.

For example, the candidate drug molecule is transformed from the SMILES format into formats such as PDB, MOL2, PDBQT, and SDF formats; a three-dimensional conformation of the candidate drug molecule is constructed based on the transformation format of the candidate drug molecule; and a hydrogen atom addible position (alkaline position) of the candidate drug molecule is determined, and a hydrogen atom is added to the hydrogen atom addible position.

Step 105. Select a target drug molecule for the target protein from the plurality of candidate drug molecules based on the activity information of each of the candidate drug molecules and the molecular docking information of each of the candidate drug molecules.

For example, after obtaining activity information of each candidate drug molecule and molecular docking information of each candidate drug molecule, drug selection is performed based on activity information and molecular docking information of each candidate drug molecule, so as to obtain a target drug molecule, thereby ensuring activity and binding inhibition performance of the target drug molecule, to provide reference data for subsequent drug molecule research and molecules and shorten the drug research period.

In some embodiments, the selecting a target drug molecule for the target protein from the plurality of candidate drug molecules based on the activity information of each of the candidate drug molecules and the molecular docking information of each of the candidate drug molecules includes: clustering the plurality of candidate drug molecules, to obtain a plurality of drug category sets; and selecting, as the target drug molecule, candidate drug molecules meeting an activity information requirement and a molecular docking information requirement from the plurality of drug category sets.

For example, candidate drug molecules are clustered, to obtain a plurality of drug category sets (clusters), and selection is performed based on the plurality of drug category sets, to obtain candidate drug molecules meeting an activity information requirement and a molecular docking information requirement to serve as the target drug molecule. Through clustering, drug molecules with structurally diversified frameworks are obtained, thereby improving diversification of the target drug molecule.

Continuing from the foregoing example, a candidate drug molecule with highest activity information or highest molecular docking information may be selected from each drug category set as a target drug molecule.

In some embodiments, the selecting candidate drug molecules meeting an activity information requirement and a molecular docking information requirement from the plurality of drug category sets to serve as the target drug molecule includes: performing the following processing for any one of the plurality of drug category sets: using a candidate drug molecule with highest activity information in the drug category set as a to-be-selected drug molecule; performing weighted summation on activity information, molecular docking information, and a drug property of the to-be-selected drug molecule, to obtain comprehensive drug information of the to-be-selected drug molecule; and ranking, based on comprehensive drug information of a plurality of to-be-selected drug molecules, the plurality of to-be-selected drug molecules in descending order, and using some top-ranked to-be-selected drug molecules of the to-be-selected drug molecules as the target drug molecules.

For example, a candidate drug molecule with highest activity information is selected from each drug category set as a to-be-selected drug molecule, activity information, molecular docking information, and a drug property (including solubility, blood-brain barrier permeability, toxicity and the like) of the to-be-selected drug molecule are determined, and a weight of the activity information, a weight of the molecular docking information and a weight of the drug property are determined; weighted summation is performed on the activity information, the molecular docking information, and the drug property of the to-be-selected drug molecule based on the weight of the activity information, the weight of the molecular docking information and the weight of the drug property, to obtain comprehensive drug information of the to-be-selected drug molecule; and based on comprehensive drug information of a plurality of to-be-selected drug molecules, the plurality of to-be-selected drug molecules are ranked in descending order, and some top-ranked to-be-selected drug molecules of the to-be-selected drug molecules are determined as the target drug molecules, thereby selecting safe target drug molecules with strong activity and a strong binding inhibition capability from numerous candidate drug molecules, to provide reference data for subsequent drug molecule research and molecules and shorten the drug research period.

The following describes an exemplary application of this embodiment of this disclosure in an actual medical treatment application scenario.

The embodiments of this disclosure may be applied to various drug molecule selection application scenarios, and a description is made below using drug molecule selection for the polycystic ovarian syndrome (PCOS) as an example:

The drug design policy in the related art needs to consume a large quantity of manpower and material resources to continuously make trial-and-error attempts. Therefore, not only the efficiency is low and the period is long, but also the accuracy is relatively low. Moreover, in this process, the blood-brain barrier permeability and other ADMET properties need to be continuously tested, so as to ensure safety and physicochemical properties of drug molecules and in-vivo metabolism stability.

To resolve the foregoing problem, the embodiments of this disclosure provide an AI-based drug virtual selection method for the polycystic ovarian syndrome (that is, AI-based drug molecule processing method), where an AI method is used for selecting 50 latent small molecule drugs (target drug molecules) having an inhibitory effect on GPR54 (target protein), that is, the AI method is used for selecting drug molecules having an inhibitory effect on the polycystic ovarian syndrome, to find and provide reference data for later development of a hit compound for treating the polycystic ovarian syndrome, and it is unnecessary to consume a large quantity of manpower and material resources to make continuously trial-and-error attempts, thereby improving molecule selection efficiency.

In this embodiment of this disclosure, information about an amino acid sequence of the GPR54 target spot is first analyzed, and the homology modeling manner is used for establishing a docking model used for further evaluating activity of small molecules. An AI algorithm model is used for learning small molecules of all GPCR target spots, to perform activity prediction scoring on the ZINC molecule library quite well, and an ADMET model is used for predicting properties of each small molecule, thereby performing selection according to ADMET properties of the molecule while ensuring activity of the molecule as much as possible.

The AI-based drug virtual selection method for the polycystic ovarian syndrome includes eight parts (selecting a to-be-selected compound library, pre-processing the compound library, DL-based compound activity scoring, establishing a molecular docking model, molecular docking scoring, post-processing selecting, compound ADMET property prediction, and final compound selection). A processing process of the AI-based drug virtual selection method for the polycystic ovarian syndrome is specifically described as follows:

### 1) selecting a to-be-selected compound library

In consideration of time and precision of molecule virtual selection, a sub-library having in-vitro activity in the ZINC library is selected, and there are a total of 276003 small molecules.

### 2) pre-processing the to-be-selected compound library

First, the selected compound library is pre-processed, which mainly includes the following processing:
1. Selection is performed based on the Lipinski's Rule of Five (Lipinski's Rule of Five, which is an empirical law of evaluating whether a compound can be used as a drug, or whether a compound having pharmacological activity or biological activity can become an oral drug), and specific selection conditions are as follows: 250 ≤ molecular weight (MW) ≤ 750; -2 ≤ logarithmic value of an oil-water partition coefficient of a compound (clogP) ≤ 7; hydrogen bond acceptor (HBA)+hydrogen bond donor (HBD)<10; the number of rotatable bonds (Num of Rotatable Bonds) <10; and topological polar surface area (TPSA) <150.
2. Deduplicating, where molecules with the same molecular structure may exist in molecules selected through the Lipinski's Rule of Five, and the molecules with the same molecular structure are deduplicated, so as to ensure that structures of the selected molecules are not the same.
3. Medicinal chemistry filter (MCF): It is used for removing molecules containing undesired groups (target groups, for example, toxicophore groups and reactive groups) in medicinal chemistry.
4. Remove an enantiomer of a probably existing chiral compound (only a SMILES structure is reserved): because the compound library contains enantiomers (between molecules forming a chiral relationship, a party is referred to as an enantiomer of the other party) of many chiral compounds (compounds that are the same in molecular weight and molecular structure, but opposite in left and right arrangement), most of small molecules may be removed through this step.

The compound library is selected from through the Lipinski's Rule of Five, to obtain 260300 molecules; 160455 small molecules not duplicate are obtained through deduplicating; and 18907 molecules are obtained by using the medicinal chemistry filter and removing enantiomers.

### 3) DL-based compound activity scoring (DL activity scoring)

As shown in FIG. 9, known sequences of all GPCR proteins and SMILES of known small molecules (candidate drug molecules) having binding activity with GPCR are used as an input, the sequences of the GPCR proteins are encoded based on a text transformer (for example, Doc-to-Vector) to obtain embedding features of the proteins, and the small molecules having binding activity with GPCR are encoded based on an image encoder (for example, DMPNN) to obtain embedding features of the small molecules; and then a binding strength of the small molecules and the GPCR proteins is predicted based on the embedding features of the small molecules and the embedding features of the proteins through a fully connected layer, and DL activity prediction is performed on the foregoing selected 18907 molecules using the model shown in FIG. 9, to obtain activity scores (which are represented by negative logarithm values pIC50 of IC50 values) respectively corresponding to the 18907 molecules.

### 4) establishing a molecular docking model

A) GPR54 homology modeling

Because the crystal structure of the GPR54 protein in the embodiments of this disclosure is unpredictable information, it is necessary to use a homology modeling method to establish a model. Structures obtained by homology modeling include a structure similar to 7TM.

### B) active amino acid (active site) and pocket detection

Most of the GPCR proteins contain many binding pockets (including active sites), and binding positions at 7TM helices are selected.

Because a definite position of an active site or a binding pocket of a ligand in the embodiments of this disclosure is unpredictable information, it is necessary to use molecular dynamics (MD) simulation (MD simulation is a comprehensive molecular simulation method integrating physics, mathematics and chemistry) to predict and confirm binding positions of small molecules and active amino acids which may form a binding pocket. A ligand-free binding protein structure is simulated through molecular dynamics, a protein is inserted into a double-layered membrane structure (POPE), a simulation space is filled with water molecules, salt ions (NaCl) with the concentration of 0.15 M are added, and simulation is performed for more than 100 ns at the temperature of 300 K in a constant pressure system. Protein structures on a simulation trajectory in last 80 ns in the foregoing simulation system are clustered, and the last clustering-center structure is taken as a molecular docking receptor structure. By using the FTMap method (that is, molecular docking method), a series of chemical groups or molecules (for example, benzene, isobutanol, uric acid and ethanol) are docked to a pocket region, and may be enriched to a region with a plurality of chemical molecules, that is, formed into a pocket region for molecular docking in a next step.

### 5) Molecular docking scoring:

Preparation is made to input a file used for docking software (for example, AutoDock Vina), and in this process, each molecularly correct molecular conformation in the compound library is determined. Charges carried by atoms and a hydrogen bond effect are quite important for successful virtual selection. The hydrogen bond effect can notably increase a binding capability of a small molecule and a protein. Therefore, whether such a protonated state of binding a protonated hydrogen atom and an atom in a particular position on a protein exists is very important. The following are steps of processing molecules in the compound library:
a. A small molecule is transformed from the SMILES format into transformation formats such as PDB, MOL2, PDBQT, and SDF formats.
b. Based on the transformation format of the small molecule, a 3D conformation of the small molecule is generated.
c. Based on the 3D conformation of the small molecule, a hydrogen atom addible position (alkaline position) of the small molecule is determined, and a hydrogen atom is added to the hydrogen atom addible position.
d. The number of charges of the small molecule with the hydrogen atom added is calculated.
e. Based on the number of charges, a correct type of the small molecule is determined.

Each of the foregoing steps is implemented by a corresponding native function module provided in the docking software (for example, AutoDock Vina).

The processed small molecule is bound to a structure obtained by the homology modeling, an energy reduced value is determined, and a larger reduced value indicates a higher molecular docking score and stronger binding.

### 6) Post-processing selection

Molecules scored through two models are clustered. To obtain molecules having structurally diversified frameworks, the foregoing 18 thousand small molecules are clustered, a total of 6446 clusters are obtained, and subsequently molecules with highest DL activity scores are selected respectively from the 6446 clusters.

The clustering is similarity-based (Tanimoto Similarity-based) clustering, and aims to improve diversification of molecules and reducing similar molecules. A similarity threshold is such set that if a similarity between molecules is greater than 0.6, the molecules are clustered to a type.

### 7) Predict ADMET properties of a compound (absorption, distribution, metabolism, excretion and toxicity of a drug)

Several relatively important basic properties of the drug molecule are selected, and in addition to the blood-brain barrier permeability (BBBP), further include the dynamics solubility (S, measuring solubleness of the drug molecule in an in-vivo environment) and toxicity. By now, 5 labels have been made for the foregoing obtained 18907 compounds respectively: DL activity score, docking score, solubility, blood-brain barrier permeability (BBBP), and toxicity. For subsequent compound selection, a compound is comprehensively selected mainly with reference to the five labels.

### 8) Final compound selection

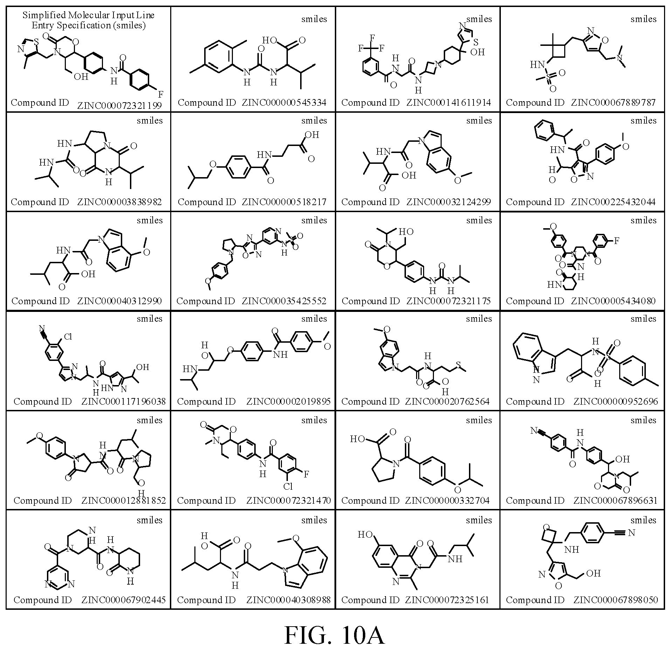

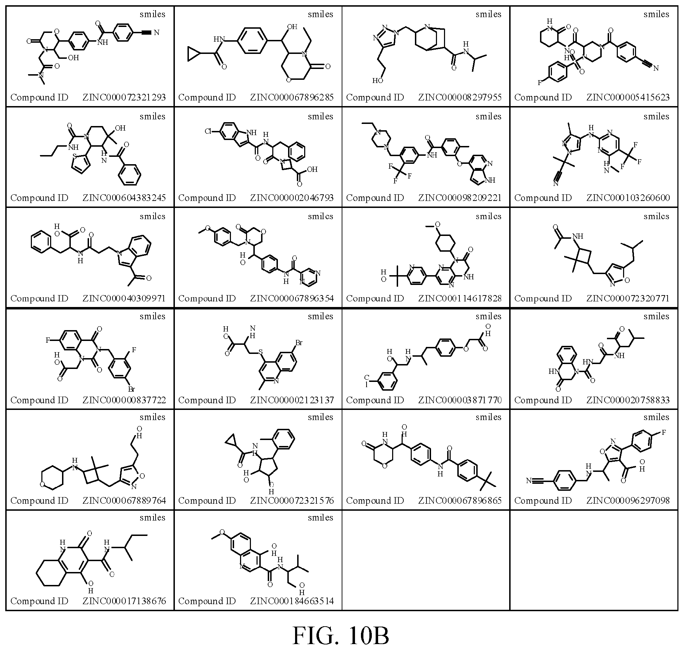

The 6446 molecules obtained by the post-processing selection are ranked in descending order according to DL activity scores (pIC50), and then latent active molecules (target drug molecules) shown in FIG. 10A and FIG. 10B are selected with reference to the solubility, the blood-brain barrier permeability, the toxicity, and the docking scores, and include 50 compounds. References of properties are as follows: the solubility log (S, mol/L) greater than -4.5 is selected, a probability value less than 0.5 is selected as BBBP, and no evident prediction toxicity is seen.

To sum up, in the AI-based drug virtual selection method for the polycystic ovarian syndrome provided in this embodiment of this disclosure, for such a chronic disease with a high incidence as the polycystic ovarian syndrome, an AI algorithm is used for selecting 50 latent drug molecules having inhibitory activity, to expand diversification of chemical structures, and further develop and provide a subsequent hit compound for this disease.

By now, the AI-based drug molecule processing method provided in the embodiments of this disclosure has been described with reference to exemplary application and implementation of the server provided in the embodiments of this disclosure. The embodiments of this disclosure further provide a drug molecule processing apparatus. During actual application, functional modules in the drug molecule processing apparatus may be cooperatively implemented by hardware resources of an electronic device (for example, terminal device, server or server cluster), for example, a calculation resource such as a processor, a communication resource (for example, being used for supporting implementation of various types of communication such as optical cable communication and cellular communication) and a memory. FIG. 4 shows the drug molecule processing apparatus 555 that is stored in the memory 550 and that may be software in the form of a program or a plug-in, for example, a software module designed by a programming language such as software C/C++ or Java, application software designed by a programming language such as C/C++ or Java, or a dedicated software module, application programming interface, plug-in, cloud service or another implementation in a large-scale software system. Different implementations are described below using examples.

### Example 1. The drug molecule processing apparatus is an application or module of a mobile terminal

The drug molecule processing apparatus 555 in the embodiments of this disclosure may be provided as a software module designed using a programming language such as software C/C++ or Java, and embedded into various applications of the mobile terminal based on Android or iOS or another system (as executable instructions stored in a storage medium of the mobile terminal and executed by a processor of the mobile terminal), thereby directly using computing resources of the mobile terminal to complete a related drug molecule selection task, and periodically or aperiodically transferring a processing result to a remote server through various network communication manners, or locally storing the processing result in the mobile terminal.

### Example 2. The drug molecule processing apparatus is an application or platform of a server

The drug molecule processing apparatus 555 in the embodiments of this disclosure may be provided as application software designed using a programming language such as C/C++ or Java or a dedicated software module in a large-scale software system, and run at a server end (as executable instructions stored in a storage medium of the server end and run by a processor of the server end), and the server uses its own computing resources to complete a related drug molecule selection task.

This embodiment of this disclosure may be further provided on a distributed and paralleled calculation platform constituted by multiple servers, and be attached a customized and easily interactive Web interface or various other user interfaces (UIs), to form a drug molecule platform (used for drug molecule selection) for a person, a group or an organization to use.

### Example 3. The drug molecule processing apparatus is an application programming interface (API) or plug-in of a server end

The drug molecule processing apparatus 555 in the embodiments of this disclosure may be provided as an API or plug-in of a server end that is invoked by a user, so as to perform the AI-based drug molecule processing method in the embodiments of this disclosure, and that is embedded into various applications.

### Example 4. The drug molecule processing apparatus is an API or plug-in of a mobile device client

The drug molecule processing apparatus 555 in the embodiments of this disclosure may be provided as an API or plug-in of a mobile device end that is invoked by a user, so as to perform the AI-based drug molecule processing method in the embodiments of this disclosure.

### Example 5. The drug molecule processing apparatus is a cloud open service

The drug molecule processing apparatus 555 in the embodiments of this disclosure may be provided as a drug molecule processing cloud service that is developed to users, for a person, a group or an organization to obtain target drug molecule.

The drug molecule processing apparatus 555 includes a series of modules, including the determining module 5551, the prediction module 5552, the processing module 5553 and the selecting module 5554. The following continues to describe that modules in the drug molecule processing apparatus 555 provided in this embodiment of this disclosure collaboratively implement a drug molecule processing solution.

The determining module 5551 is configured to determine a plurality of candidate drug molecules for a target protein; the prediction module 5552 is configured to perform activity prediction based on the plurality of candidate drug molecules and the target protein, to obtain activity information of each of the candidate drug molecules; the processing module 5553 is configured to perform homology modeling on the target protein, to obtain a reference protein having a structure homologous with that of the target protein; and perform molecular docking based on the reference protein and the plurality of candidate drug molecules, to obtain molecular docking information of each of the candidate drug molecules; and the selecting module 5554 is configured to select from the plurality of candidate drug molecules based on the activity information of each of the candidate drug molecules and the molecular docking information of each of the candidate drug molecules, to obtain target drug molecule for the target protein.

In some embodiments, the determining module 5551 is further configured to select a plurality of compounds from compounds in a compound library based on the target protein; and pre-process the plurality of selected compounds, to obtain the candidate drug molecules for the target protein.

In some embodiments, the determining module 5551 is further configured to perform Lipinski's Rule of Five-based selection on the compounds in the compound library based on the target protein, to obtain a plurality of compounds obeying the Lipinski's Rule of Five; and deduplicate the plurality of compounds obeying the Lipinski's Rule of Five, to obtain the plurality of selected compounds.

In some embodiments, the determining module 5551 is further configured to chemically filter the plurality of selected compounds based on a target group, to obtain a plurality of filtered compounds; and removing an enantiomer of a chiral compound from the plurality of filtered compounds, to obtain the candidate drug molecules for the target protein.

In some embodiments, the prediction module 5552 is further configured to perform the following processing for any one of the plurality of candidate drug molecules: encoding a molecular structure of the candidate drug molecule, to obtain an embedding feature of the candidate drug molecule; encoding a protein structure of the target protein, to obtain an embedding feature of the target protein; fusing the embedding feature of the candidate drug molecule and the embedding feature of the target protein, to obtain an activity fusion feature; and mapping the activity fusion feature, to obtain activity information of the candidate drug molecule.

In some embodiments, the prediction module 5552 is further configured to determine a molecular graph of the candidate drug molecule based on the molecular structure of the candidate drug molecule; and perform image encoding on the molecular graph of the candidate drug molecule, to obtain the embedding feature of the candidate drug molecule.

In some embodiments, the prediction module 5552 is further configured to determine a protein sequence of the target protein based on the protein structure of the target protein; and perform text transformation on the protein sequence of the target protein, to obtain the embedding feature of the target protein.

In some embodiments, the prediction module 5552 is further configured to sum the embedding feature of the candidate drug molecule and the embedding feature of the target protein, to obtain the activity fusion feature; or concatenate the embedding feature of the candidate drug molecule and the embedding feature of the target protein, to obtain the activity fusion feature.

In some embodiments, the prediction module 5552 is further configured to map the embedding feature of the candidate drug molecule and the embedding feature of the target protein, to obtain an intermediate feature vector including the candidate drug molecule and the target protein; and perform affine transformation on the intermediate feature vector, to obtain the activity fusion feature.

In some embodiments, the prediction module 5552 is further configured to map the activity fusion feature to a latent vector space, to obtain a latent vector of the activity fusion feature; and perform nonlinear mapping on the latent vector of the activity fusion feature, and use an obtained activity mark of the candidate drug molecule as the activity information of the candidate drug molecule.

In some embodiments, the processing module 5553 is further configured to perform the following processing for any candidate protein in a protein library: performing similarity processing on a sequence of the candidate protein and a sequence of the target protein, to obtain a similarity between the candidate protein and the target protein; and performing structure optimization based on a three-dimensional structure of the candidate protein in a case that the similarity is greater than a similarity threshold, to obtain the reference protein having the structure homologous with that of the target protein.

In some embodiments, the processing module 5553 is further configured to perform molecular dynamics simulation based on the reference protein, to obtain an active site and a binding pocket of the reference protein; pre-process the plurality of candidate drug molecules respectively, to obtain a molecular conformation of each of the candidate drug molecules; and perform the following processing for the molecular conformation of each of the candidate drug molecules: performing molecular docking scoring based on the active site and the binding pocket of the reference protein and the molecular conformation of the candidate drug molecule, and using a result of the molecular docking scoring as the molecular docking information of the candidate drug molecule.

In some embodiments, the processing module 5553 is further configured to perform format transformation on the plurality of candidate drug molecules respectively, to obtain a transformation format of each of the candidate drug molecules; construct a three-dimensional conformation of each of the candidate drug molecules based on the transformation format of each of the candidate drug molecules; determine a hydrogen atom addible position of each of the candidate drug molecules based on the three-dimensional conformation of each of the candidate drug molecules; and add a hydrogen atom to the hydrogen atom addible position, to obtain the molecular conformation of the candidate drug molecule.

In some embodiments, the selecting module 5554 is further configured to cluster the plurality of candidate drug molecules, to obtain a plurality of drug category sets; and select, as the target drug molecule, candidate drug molecules meeting an activity information requirement and a molecular docking information requirement from the plurality of drug category sets.

In some embodiments, the selecting module 5554 is further configured to perform the following processing for any one of the plurality of drug category sets: using a candidate drug molecule with highest activity information in the drug category set as a to-be-selected drug molecule; performing weighted summation on activity information, molecular docking information, and a drug property of the to-be-selected drug molecule, to obtain comprehensive drug information of the to-be-selected drug molecule; and ranking, based on comprehensive drug information of a plurality of to-be-selected drug molecules, the plurality of to-be-selected drug molecules in descending order, and using some top-ranked to-be-selected drug molecules of the to-be-selected drug molecules as the target drug molecule.

An embodiment of this disclosure provides a computer program product or a computer program, the computer program product or the computer program including computer instructions, the computer instructions being stored in a computer-readable storage medium. A processor of a computer device reads the computer instructions from the computer-readable storage medium and executes the computer instructions to cause the computer device to perform the foregoing AI-based drug molecule processing method in the embodiments of this disclosure.

An embodiment of this disclosure provides a storage medium storing an executable instruction. When the executable instruction is executed by a processor, the processor is caused to perform an AI-based questions and answers processing method provided in the embodiments of this disclosure, for example, the AI-based drug molecule processing methods shown in FIG. 5 to FIG. 7.

In some embodiments, the computer-readable storage medium may be a memory such as an FRAM, a ROM, a PROM, an EPROM, an EEPROM, a flash memory, a magnetic surface memory, an optical disk, or a CD-ROM, or may be any device including one of or any combination of the foregoing memories.

In some embodiments, the executable instructions can be written in a form of a program, software, a software module, a script, or code and according to a programming language (including a compiler or interpreter language or a declarative or procedural language) in any form, and may be deployed in any form, including an independent program or a module, a component, a subroutine, or another unit suitable for use in a computing environment.

In an example, the executable instructions may, but do not necessarily, correspond to a file in a file system, and may be stored in a part of a file that saves another program or other data, for example, be stored in one or more scripts in a HyperText Markup Language (HTML) file, stored in a file that is specially used for a program in discussion, or stored in the plurality of collaborative files (for example, be stored in files of one or modules, subprograms, or code parts).

In an example, the executable instructions may be deployed to be executed on a computing device, or deployed to be executed on a plurality of computing devices at the same location, or deployed to be executed on a plurality of computing devices that are distributed in a plurality of locations and interconnected by using a communication network.

The foregoing descriptions are merely embodiments of this disclosure and are not intended to limit the protection scope of this disclosure. Any modification, equivalent replacement, or improvement made without departing from the spirit and range of this disclosure shall fall within the protection scope of this disclosure.

## Claims

1. An artificial intelligence (AI)-based drug molecule processing method, applicable to an electronic device, the method comprising:
determining a plurality of candidate drug molecules for a target protein;
performing activity prediction based on the plurality of candidate drug molecules and the target protein, to obtain activity information of each of the plurality of candidate drug molecules;
performing homology modeling on the target protein, to obtain a reference protein having a structure homologous with that of the target protein;
performing molecular docking between the reference protein and the plurality of candidate drug molecules, to obtain molecular docking information of each of the plurality of candidate drug molecules; and
selecting a target drug molecule for the target protein from the plurality of candidate drug molecules based on the activity information of each of the candidate drug molecules and the molecular docking information of each of the candidate drug molecules.

2. The method according to claim 1, wherein the determining a plurality of candidate drug molecules for a target protein comprises:
selecting a plurality of compounds from compounds in a compound library based on the target protein; and
pre-processing the plurality of selected compounds, to obtain the plurality of candidate drug molecules for the target protein.

3. The method according to claim 2, wherein the selecting a plurality of compounds from compounds in a compound library based on the target protein comprises:
performing Lipinski's Rule of Five-based selection on the compounds in the compound library based on the target protein, to obtain a plurality of compounds obeying the Lipinski's Rule of Five; and
deduplicating the plurality of compounds obeying the Lipinski's Rule of Five, to obtain the plurality of selected compounds.

4. The method according to claim 2, wherein the pre-processing the plurality of selected compounds, to obtain the candidate drug molecules for the target protein comprises:
chemically filtering the plurality of selected compounds based on a target group, to obtain a plurality of filtered compounds; and
removing an enantiomer of a chiral compound from the plurality of filtered compounds, to obtain the plurality of candidate drug molecules for the target protein.

5. The method according to claim 1, wherein the performing activity prediction based on the plurality of candidate drug molecules and the target protein, to obtain activity information of each of the candidate drug molecules comprises:
performing the following processing for any one of the plurality of candidate drug molecules:
encoding a molecular structure of the candidate drug molecule, to obtain an embedding feature of the candidate drug molecule;
encoding a protein structure of the target protein, to obtain an embedding feature of the target protein;
fusing the embedding feature of the candidate drug molecule and the embedding feature of the target protein, to obtain an activity fusion feature; and
mapping the activity fusion feature, to obtain activity information of the candidate drug molecule.

6. The method according to claim 5, wherein the encoding a molecular structure of the candidate drug molecule, to obtain an embedding feature of the candidate drug molecule comprises:
determining a molecular graph of the candidate drug molecule based on the molecular structure of the candidate drug molecule; and
performing image encoding on the molecular graph of the candidate drug molecule, to obtain the embedding feature of the candidate drug molecule.

7. The method according to claim 5, wherein the encoding a protein structure of the target protein, to obtain an embedding feature of the target protein comprises:
determining a protein sequence of the target protein based on the protein structure of the target protein; and
performing text transformation on the protein sequence of the target protein, to obtain the embedding feature of the target protein.

8. The method according to claim 5, wherein the fusing the embedding feature of the candidate drug molecule and the embedding feature of the target protein, to obtain an activity fusion feature comprises:
summing the embedding feature of the candidate drug molecule and the embedding feature of the target protein, to obtain the activity fusion feature; or
concatenating the embedding feature of the candidate drug molecule and the embedding feature of the target protein, to obtain the activity fusion feature.

9. The method according to claim 5, wherein the fusing the embedding feature of the candidate drug molecule and the embedding feature of the target protein, to obtain an activity fusion feature comprises:
mapping the embedding feature of the candidate drug molecule and the embedding feature of the target protein, to obtain an intermediate feature vector comprising the candidate drug molecule and the target protein; and
performing affine transformation on the intermediate feature vector, to obtain the activity fusion feature.

10. The method according to claim 5, wherein the mapping the activity fusion feature, to obtain activity information of the candidate drug molecule comprises:
mapping the activity fusion feature to a latent vector space, to obtain a latent vector of the activity fusion feature; and
performing nonlinear mapping on the latent vector of the activity fusion feature, to obtain the activity information of the candidate drug molecule.

11. The method according to claim 1, wherein the performing homology modeling on the target protein, to obtain a reference protein having a structure homologous with that of the target protein comprises:
performing the following processing for any candidate protein in a protein library:
performing similarity processing on a sequence of the candidate protein and a sequence of the target protein, to obtain a similarity between the candidate protein and the target protein; and
performing structure optimization based on a three-dimensional structure of the candidate protein in responsive to the similarity being greater than a similarity threshold, to obtain the reference protein having the structure homologous with that of the target protein.

12. The method according to claim 1, wherein the performing molecular docking between the reference protein and the plurality of candidate drug molecules, to obtain molecular docking information of each of the candidate drug molecules comprises:
performing molecular dynamics simulation based on the reference protein, to obtain an active site and a binding pocket of the reference protein;
pre-processing the plurality of candidate drug molecules respectively, to obtain a molecular conformation of each of the plurality of candidate drug molecules; and
performing the following processing for the molecular conformation of each of the plurality of candidate drug molecules: performing molecular docking scoring based on the active site and the binding pocket of the reference protein and the molecular conformation of the candidate drug molecule, and using a result of the molecular docking scoring as the molecular docking information of the candidate drug molecule.

13. The method according to claim 12, wherein the pre-processing the plurality of candidate drug molecules respectively, to obtain a molecular conformation of each of the candidate drug molecules comprises:
performing format transformation on the plurality of candidate drug molecules respectively, to obtain a transformation format of each of the plurality of candidate drug molecules;
constructing a three-dimensional conformation of each of the plurality of candidate drug molecules based on the transformation format of each of the plurality of candidate drug molecules;
determining a hydrogen atom addible position of each of the plurality of candidate drug molecules based on the three-dimensional conformation of each of the plurality of candidate drug molecules; and
adding a hydrogen atom to the hydrogen atom addible position, to obtain the molecular conformation of the candidate drug molecule.

14. The method according to claim 1, wherein the selecting a target drug molecule for the target protein from the plurality of candidate drug molecules based on the activity information of each of the candidate drug molecules and the molecular docking information of each of the candidate drug molecules comprises:
clustering the plurality of candidate drug molecules, to obtain a plurality of drug category sets; and
selecting, as the target drug molecule, candidate drug molecules meeting an activity information requirement and a molecular docking information requirement from the plurality of drug category sets.

15. The method according to claim 14, wherein the selecting candidate drug molecules meeting an activity information requirement and a molecular docking information requirement from the plurality of drug category sets to serve as the target drug molecule comprises:
performing the following processing for any one of the plurality of drug category sets:
using a candidate drug molecule with highest activity information in the drug category set as a to-be-selected drug molecule;
performing weighted summation on activity information, molecular docking information, and a drug property of the to-be-selected drug molecule, to obtain comprehensive drug information of the to-be-selected drug molecule; and
ranking, based on comprehensive drug information of a plurality of to-be-selected drug molecules, the plurality of to-be-selected drug molecules in descending order, and using some top-ranked to-be-selected drug molecules of the to-be-selected drug molecules as the target drug molecule.

16. An artificial intelligence (AI)-based drug molecule processing apparatus, comprising:
a determining module, configured to determine a plurality of candidate drug molecules for a target protein;
a prediction module, configured to perform activity prediction based on the plurality of candidate drug molecules and the target protein, to obtain activity information of each of the plurality of candidate drug molecules;
a processing module, configured to perform homology modeling on the target protein, to obtain a reference protein having a structure homologous with that of the target protein; and perform molecular docking between the reference protein and the plurality of candidate drug molecules, to obtain molecular docking information of each of the plurality of candidate drug molecules; and
a selecting module, configured to select a target drug molecule for the target protein from the plurality of candidate drug molecules based on the activity information of each of the candidate drug molecules and the molecular docking information of each of the candidate drug molecules.

17. An electronic device, comprising:
a memory, configured to store executable instructions; and
a processor, configured to perform the artificial intelligence-based (AI-based) drug molecule processing method according to any one of claims 1 to 15 when executing the executable instructions stored in the memory.

18. A computer-readable storage medium, storing executable instructions, the executable instructions, when executed by a processor, implementing the artificial intelligence-based (AI-based) drug molecule processing method according to any one of claims 1 to 15.

19. A computer program product, comprising a computer program or instruction, the computer program or instruction causing a computer to perform the artificial intelligence (AI)-based drug molecule processing method according to any one of claims 1 to 15.
